(19) Europäisches Patentamt
European Patent Office
Office européen des brevets

(11) **EP 0 552 463 B1**

(12) **EUROPÄISCHE PATENTSCHRIFT**

(45) Veröffentlichungstag und Bekanntmachung des Hinweises auf die Patenterteilung:
**17.04.1996 Patentblatt 1996/16**

(51) Int. Cl.$^6$: **B01J 23/84**, C07C 29/149, C07C 29/145, C07C 29/141

(21) Anmeldenummer: **92121204.9**

(22) Anmeldetag: **12.12.1992**

(54) **Chromfreier Katalysator für die Hydrierung von organischen Verbindungen**

Chromium-free catalyst for the selective hydrogenation of organic compounds

Catalyseur exempt de chrome pour l'hydrogénation sélective de composés organiques

(84) Benannte Vertragsstaaten:
**BE DE DK FR GB IT NL**

(30) Priorität: **13.12.1991 DE 4141199**

(43) Veröffentlichungstag der Anmeldung:
**28.07.1993 Patentblatt 1993/30**

(73) Patentinhaber: **SÜD-CHEMIE AG**
**D-80333 München (DE)**

(72) Erfinder:
• **Schneider, Michael, Dr.**
**W-8012 Ottobrunn (DE)**
• **Kochloefl, Karl, Dr.**
**W-8206 Bruckmühl (DE)**
• **Maletz, Gerd J., Dr.**
**W-8206 Bruckmühl (DE)**

(74) Vertreter: **Reitzner, Bruno, Dr. et al**
**Patentanwälte Dipl.-Ing. R. Splanemann**
**Dr. B. Reitzner, Dipl.-Ing. K. Baronetzky**
**Tal 13**
**D-80331 München (DE)**

(56) Entgegenhaltungen:
EP-A- 0 034 338          EP-A- 0 300 346
EP-A- 0 434 062          DE-A- 1 248 623
GB-A- 2 025 252

## Beschreibung

Die Erfindung betrifft einen chromfreien Katalysator für die Hydrierung von organischen Verbindungen, insbesondere von organischen Verbindungen, die die Carbonylfunktion enthalten. Der Katalysator ist insbesondere für die Hydrierung von Aldehyden, Ketonen sowie von Carbonsäuren bzw. deren Estern, speziell von Fettsäuren bzw. deren Estern, zu den entsprechenden Alkoholen geeignet.

Fettalkohole, d.h. aliphatische, vorwiegend lineare, primäre Alkohle mit Kettenlängen von mehr als 8 Kohlenstoffatomen stellen bedeutende Zwischenprodukte der chemischen Industrie dar. Verwendung finden sie bevorzugt zur Herstellung von Tensiden wie z.B. Fettalkoholsulfaten, Polyglykolethern oder Polyglykolethersulfaten.

Wichtiger Rohstoff für ihre Produktion sind Fettsäuren bzw. Fettsäureester, wie sie als Gemische unterschiedlicher Kettenlängen u.a. aus natürlichen Fetten und Ölen erhalten werden können. Die Überführung in die Fettalkohole erfolgt durch katalytische Druckhydrierung, wobei sich vor allem Katalysatoren auf Basis Kupfer-Chrom bewährt haben.

Die Hydrierungsreaktion wird als Suspensionshydrierung, als Gasphasenhydrierung oder in der Rieselphase durchgeführt. Ausreichend hohe Reaktionsgeschwindigkeiten werden erst bei Drükken oberhalb 250 bar und Temperaturen im Bereich 260 bis 300°C erreicht. In der Regel werden die Triglyceride vor der Hydrierung nach bekannten Methoden mit Methanol umgeestert, und die freien Fettsäuren werden verestert. Dennoch weist das Reaktionsgemisch eine Restkonzentration an freien Carbonsäuren auf.

Eine wichtige technische Ausführungsform stellt die veresternde Suspensionshydrierung von Fettsäuren nach dem LURGI- Verfahren dar. Bei diesem Verfahren wird das Fettsäurengemisch dem Hydrierungsreaktor kontinuierlich zugeführt und in situ mit im Überschuß vorhandenem Fettalkohol verestert.

Die Anwesenheit der freien Fettsäuren stellt offensichtlich hohe Anforderungen an die Säurebeständigkeit der verwendeten Katalysatoren. Durch den Angriff der Säuren können die Katalysatormetalle, insbesondere Kupfer, eluiert werden, so daß der Katalysator in seiner Wirksamkeit beeinträchtigt wird. Darüber hinaus kommt es auch zur Verunreinigung des Produkts durch Metallseifen bzw. zur Abscheidung von Cu-haltigem Material im Hydrierreaktor bzw. in nachgeschalteten Anlagenteilen.

Die gegenwärtig verwendeten Cu-Cr-Oxid Katalysatoren weisen zufriedenstellende Hydrieraktivität und ausreichende Beständigkeit gegen die im Reaktionsgemisch vorhandenen Fettsäuren auf. Ein großer Nachteil dieser Katalysatoren liegt aber in ihrer chemischen Zusammensetzung. Wie alle Katalysatoren verlieren sie mit der Zeit unter der Einwirkung der im zu hydrierenden Substrat enthaltenen Katalysatorgifte ihre Wirksamkeit und müssen entsorgt werden. Chromhaltige Altkatalysatoren sind, insbesondere wenn ein Gehalt an sechswertigem Chrom nicht ausgeschlossen werden kann, als umweltgefährdende Stoffe zu betrachten. Die Entsorgung stellt die Betreiber von Fettalkoholanlagen in zunehmdem Maße vor Probleme und verursacht nicht zuletzt die Wirtschaftlichkeit des Verfahrens belastende Kosten.

Auch bei der Hydrierung von Aldehyden und Ketonen kann ein gewisser Gehalt an aciden Komponenten zu einer Verschlechterung der Katalysatoreigenschaften führen.

Aus der EP-A-0434062 sind Katalysatoren für die Hydrierung von sauerstoffhaltigen Verbindungen, z.B. von Carbonsäureestern in der Dampfphase bekannt, die Kupferoxid und Aluminiumoxid als Hauptbestandteile und daneben u.a. Zirkonoxid enthalten. Die oxidischen Vorstufen dieser Katalysatoren werden bei maximal 550°C calciniert, so daß die Säurebeständigkeit gering ist. Außerdem ist die Aktivität der Katalysatoren relativ gering.

Aus der SU-A-39 90 97 ist ein Katalysator für die Umsetzung von Kohlenoxiden mit Wasserdampf bekannt, der Kupferoxid, Zinkoxid und Aluminiumoxid enthält. Für Katalysatoren für die Hydrierung von höheren organischen Verbindungen, die die Carbonylfunktion enthalten, wird kein Zinkoxid benötigt.

Aus der SU-A-38 22 61 ist ein Katalysator für die Umsetzung von Kohlenoxid mit Wasserdampf auf der Basis von Kupferoxid und Oxiden von dreiwertigen Metallen bekannt, wobei als dreiwertige Metalle Aluminium, Chrom, Mangan und Eisen in Frage kommen. Chrom ist ein wesentlicher Bestandteil dieser Katalysatoren.

Aus der DE-B-1 248 623 ist ein Verfahren zur Herstellung von Wasserstoff durch Umsetzung von Kohlenoxid oder kohlenoxidhaltigen Gasen mit Wasserdampf bei erhöhter Temperatur an Katalysatoren auf der Basis von Kupferoxid, Aluminiumoxid und/oder Chrom- und/oder Manganoxid bekannnt, wobei die Katalysatoren nach allen Beispielen Chromoxid enthalten. Das Verfahren hat keine Berührungspunkte mit dem Verfahren, für das die erfindungsgemäßen Katalysatoren verwendet werden sollen.

Aus der DD-A-242 183 ist ein Verfahren zur Herstellung eines Oxidationskatalysators bekannt, der aus Kupfernitrat, Mangannitrat und Aluminiumnitrat durch Fällung mit einer wäßrigen Natriumcarbonatlösung als Vorkatalysator hergestellt wird; dieser wird gewaschen, getrocknet und geglüht. Der Aluminiumoxidgehalt dieses Katalysators liegt bei mehr als 70 Gew.%.

Aus der EP-A-0 434 062 sind Hydrierkatalysatoren auf der Basis von Kupfer, Aluminium und einem Metall aus der Gruppe Magnesium, Zink, Titan, Zirkon, Zinn, Nickel, Cobalt oder deren Gemischen bekannt. Die Katalysatoren enthalten kein Mangan.

Aus der DE-A-4 021 230 ist ein Verfahren zur Herstellung von Alkoholen unter Verwendung von Kupfer-Zirkonoxid-Katalysatoren bekannt. Diese Katalysatoren enthalten kein Magnesium oder Aluminium.

Aus der DE-A-4 028 295 sind Kupfer-Mangan-Katalysatoren für die Hydrierung von Fettsäuren, Fettsäureglyceridestern oder Fettsäureniedirgalkylestern zu Fettalkoholen entsprechender Kettenlänge bekannt. Diese Katalysatoren enthalten kein Aluminium.

Aufgabe der vorliegenden Erfindung war es, Katalysatoren für die Hydrierung von organischen Verbindungen, insbesondere von Verbindungen, die die Carbonylfunktion enthalten, z.B. von Aldehyden, Ketonen oder von Carbonsäuren bzw. deren Estern bereitzustellen, die hohe Hydrieraktivität sowie gute Beständigkeit gegenüber aciden Komponenten besitzen, gleichzeitig aber keinerlei umweltschädigende Wirkung haben.

Diese Aufgabe konnte gelöst werden durch einen chromfreien Katalysator, der in einer ersten Ausführungsform durch folgende Merkmale gekennzeichnet ist:

a) seine oxidische Form entspricht im wesentlichen der Zusammensetzung

$$Cu_aAl_bZr_cMn_dO_x$$

wobei die folgenden Beziehungen gelten:
$a > O; b > O; c \geq O; d > O; a > b/2; b > a/4; a > c; a > d;$
und x die zur Wahrung der Elektroneutralität pro Formeleinheit erforderliche Anzahl von Sauerstoffionen bezeichnet;

b) die Metallmasse, welche durch 2-minütiges Rühren von 10 g des oxidischen Katalysators in 100 ml 10 gew.%-iger Essigsäure bei 200 °C gelöst wird, beträgt maximal 400 mg.

c) die BET-Oberfläche liegt zwischen 10 und 150 m²/g.

Nach einer weiteren Ausführungsform enthält der erfindungsgemäße Katalysator einen geringeren Anteil an Aluminiumoxid. Der Katalysator nach dieser Ausführungsform ist durch folgende Merkmale gekennzeichnet:

a) seine oxidische Form entspricht im wesentlichen der Zusammensetzung

$$Cu_aAl_bZr_cMn_dO_x$$

wobei die folgenden Beziehungen gelten:
$a > O; b = a/40$ bis $a/4; c \geq O; d > O; a > c; a = 0,5d$ bis $0,95d$
und x die zur Wahrung der Elektroneutralität pro Formeleinheit erforderliche Anzahl von Sauerstoffionen bezeichnet;

b) die Metallmasse, welche durch 2-minütiges Rühren von 10 g des oxidischen Katalysators in 100 ml 10 gew.%-iger Essigsäure bei 200 °C gelöst wird, beträgt maximal 400 mg;

c) die BET-Oberfläche liegt zwischen 5 und 150 m²/g.

d) die oxidische Form ist durch thermische Behandlung einer Zwischenstufe im Temperaturbereich von 450 bis 900 °C, vorzugsweise im Temperaturbereich von 500 bis 800 °C, erhältlich.

Im Zusammenhang mit der Entwicklung der zweiten Ausführungsform wurde festgestellt, daß sich die Lebensdauer des Katalysators verbessert, wenn der Anteil des Aluminiumoxids vermindert wird. Der Chemismus dieses Effekts ist noch nicht in allen Details geklärt, doch gibt es Anzeichen dafür, daß in diesem Zusammenhang Rehydratisierungsphänomenen eine maßgebliche Rolle zufällt. Wird der Anteil des Aluminiumoxids vermindert, so ist zur Erhöhung der Säurebeständigkeit die vorstehend angegebene thermische Behandlung der oxidischen Vorstufe erforderlich. Durch die Verminderung des Aluminiumgehalts vermindert sich im allgemeinen auch die BET-Oberfläche.

Vorzugsweise ist der Anteil des Mangans bei beiden Ausführungsformen höher als der Anteil des Zirkons, d.h. $d > c$.

Die BET-Oberfläche des Katalysators nach der ersten Ausführungsform liegt vorzugsweise zwischen 25 und 125 m²/g, die nach der zweiten Ausführungsform vorzugsweise zwischen 5 und 100 m²/g.

Die erfindungsgemäßen Katalysatoren können auch ein inertes Trägermaterial, wie $SiO_2$, $TiO_2$ und deren Gemische enthalten.

Die Herstellung der erfindungsgemäßen Katalysatoren kann im Prinzip nach den bekannten Methoden, die zur Herstellung einer für katalytische Anwendungen ausreichend intensiven Mischung der durch thermische Behandlung in die Oxide überführbaren Zwischenstoffe geeignet sind, erfolgen.

Bevorzugt wird die Fällung von schwerlöslichen Verbindungen aus Lösungen, welche die entsprechenden Metallionen in Form ihrer Salze enthalten. Geeignete Salze sind z.B. die Halogenide, die Sulfate und die Nitrate. Die Zirkonkomponente (falls vorhanden) wird bevorzugt in Form saurer Zirkonylsalzlösungen verwendet, kann aber auch durch Hydrolyse ammoniakalischer $NH_4$-Zirkonylcarbonatlösungen freigesetzt werden.

Als Fällungsmittel eignen sich alle Agenzien, die zur Bildung solcher unlöslicher Zwischenstufen führen, die sich durch thermische Behandlung in die Oxide überführen lassen. Besonders geeignete Zwischenstufen sind die Hydroxide und Carbonate bzw. Hydroxocarbonate, so daß man als besonders bevorzugte Fällungsmittel Alkalicarbonate oder Ammoniumcarbonat einsetzt.

Als besonders geeignete Methode zur Herstellung der Zwischenstufen ist die Copräzipitation anzusehen.

Das Auswaschen der erhaltenen Niederschläge ist nicht besonders kritisch, d.h. die Katalysatoren können noch einen gewissen Restalkaligehalt enthalten, ohne daß ihre Wirkungsweise beeinträchtigt wird. In gewissen Fällen wurde sogar eine Aktivitätssteigerung beobachtet.

Außerdem können Promotoren, insbesondere solche, die die Acidität der Aluminiumoxidkomponente vermindern, wie z.B. Erdalkioxide oder Seltenerdoxide, in Mengen von 0,1 bis 20 Gew.-% (berechnet als Oxide und bezogen auf die oxidische Form des Katalysators), verwendet werden.

Wichtig für die Herstellung der erfindungsgemäßen Katalysatoren, insbesondere nach der zweiten Ausführungsform, ist die thermische Behandlung der Zwischenstufen, wobei nach der ersten Ausführungsform vorzugsweise im Temperaturbereich zwischen 500 und 1000 °C, insbesondere im Temperaturbereich zwischen 600 und 900 °C gearbeitet wird. Der bevorzugte Temperaturbereich bei der zweiten Ausführungsform liegt zwischen 500 und 600 °C.

Wird diese thermische Behandlung bei zu niedriger Temperatur durchgeführt, so ist die Stabilisierung des Kupferoxids in der Matrix nicht ausreichend, und es kommt während der Hydrierungsreaktion zu einem Herauslösen der Cu-Komponente bzw. auch der übrigen Metallbestandteile des Katalysators unter der Einwirkung der im Reaktionsgemisch anwesenden aciden Komponente mit den geschilderten negativen Folgen für die Wirksamkeit des Katalysators wie auch für das Produkt bzw. den Reaktor und nachgeschaltete Anlagenteile.

Die thermische Behandlung kann diskontinuierlich oder kontinuierlich, z.B. im Drehrohrofen durchgeführt werden. Der Katalysator kann in der oxidischen Form oder in vorreduzierter Form in den Handel gebracht werden. Gegenstand der Erfindung ist somit auch die vorreduzierte Form des Katalysators. Die Vorreduzierung erfolgt in an sich bekannter Weise durch Erhitzen der oxidischen Form des Katalysators in einer reduzierenden Atmosphäre, und zwar im allgemeinen bei Temperaturen von 150 bis 450 °C. Der Katalysator kann aber auch in situ unter den bei der Hydrierung vorgegebenen Bedingungen, vorzugsweise unter Druck (z.B. bei einem Wasserstoffdruck von etwa 300 bar) reduziert werden.

Gegenstand der Erfindung ist auch die Verwendung des vorstehend beschriebenen Katalysators zur Hydrierung von organischen Verbindungen. Außer zur Hydrierung von ungesättigten organischen Verbindungen werden die Katalysatoren insbesondere zur Hydrierung von organischen Verbindungen, die die Carbonylfunktion enthalten, z.B. von Aldehyden, Ketonen, sowie von Carbonsäuren bzw. deren Estern, zu den entsprechenden Alkoholen, verwendet. Die Verbindungen, die die Carbonylfunktion enthalten, können ansonsten in der Kette gesättigt oder einfach bzw. mehrfach ungesättigt sein.

Insbesondere eignet sich der erfindungsgemäße Katalysator zur Sumpfphasenhydrierung von Carbonsäuren, insbesondere von Fettsäuren bzw. Fettsäuregemischen mit 5 bis 24 C-Atomen und/oder deren Estern, gegebenenfalls im Gemisch mit Alkoholen, in die entsprechenden Fettalkohole. Hierbei können die Fettsäuren bzw. Fettsäuregemische in situ mit im Reaktionsgemisch vorhandenen Alkoholen verestert werden. Bevorzugte, im Reaktionsgemisch vorhandene Alkohole sind Fettalkohole oder Gemische von Fettalkoholen mit 4 bis 25 C-Atomen.

Zur Sumpfphasenhydrierung werden die Katalysatoren als Pulver eingesetzt. Bei der Dampfphasenhydrierung, sowie bei der Hydrierung der Rieselphase (insbesondere von Carbonsäurealkylestern) werden die Katalysatoren als Formkörper eingesetzt, z.B. in Form von gepressten Zylindern, Tabletten, Pastillen, Wagenrädern, Ringen, Sternen oder Strangpreßlingen, wie Vollsträngen, polylobären Strängen, Hohlsträngen und Wabenkörpern.

Die Erfindung ist durch die nachstehenden Beispiele erläutert, wobei sich die Beispiele 1 bis 4 auf die Herstellung des Katalysators nach der ersten Ausführungsform und die Beispiele 5 bis 8 auf die Herstellung des Katalysators nach der zweiten Ausführungsform beziehen. Beispiel 9 bezieht sich auf die Anwendung der erfindungsgemäßen Katalysatoren.

Beispiel 1

Man löst 484 g $Cu(NO_3)_2*3\ H_2O$, 750 g $Al(NO_3)_3*9H_2O$ sowie 100 g $Mn(NO_3)_2*4H_2O$ in entsalztem Wasser auf 2 Liter Gesamtvolumen und erwärmt die Lösung auf 60 °C. Die erhaltene Lösung wird nun während einer Stunde in einen gerührten Fällbehälter gepumpt. Gleichzeitig wird ebenfalls auf 60°C erhitzt 1.5 molare Sodalösung mit einer solchen Dosierrate zugeführt, daß der pH-Wert im Fällbehälter 6.2 + 0.2 beträgt. Nach Beendigung der Fällung wird noch eine weitere Stunde bei 60°C gerührt. Man filtriert und wäscht den Filterkuchen. Getrocknet wird über Nacht bei 120°C. Anschließend wird wie folgt calciniert: man heizt mit einer Heizrate von 2°/min auf 600°C und beläßt es 3 h bei dieser Temperatur. Die BET-Oberfläche wurde über die $N_2$-Sorption nach der Einpunkt-Methode gemäß DIN 66 132 zu 110 $m^2/g$ bestimmt.

4

Beispiel 2

Man verfährt, wie im Beispiel 1 beschrieben, bis zur Trocknung des erhaltenen Filterkuchens, führt aber die Calcinierung folgendermaßen durch: Aufheizen mit 2°/min auf 800°C und Halten dieser Temperatur für einen Zeitraum von 3 Stunden. Die BET-Oberfläche beträgt 69 m²/g.

Beispiel 3

Man stellt eine Cu-Al-Mn-Zr-Nitratlösung her durch Lösen von 484 g $Cu(NO_3)_2$*$3H_2O$, 750 g Al $(NO_3)_3$*$9H_2O$, 50 g $Mn(NO_3)_2$*$4H_2O$ sowie 123 g einer Zirkonylnitratlösung enthaltend 20 % $ZrO_2$ auf ein Gesamtvolumen von 2 Liter. Fällung und Aufarbeitung einschließlich der Calcinierung bei einer Maximaltemperatur von 600°C werden wie im Beispiel 1 beschrieben, vorgenommen. Die BET-Oberfläche wurde zu 116 m²/g ermittelt.

Beispiel 4

Man verfährt, wie im Beispiel 3 beschrieben, bis zur Trocknung des erhaltenen Filterkuchens, führt aber die Calcinierung folgendermaßen durch: Aufheizen mit 2°/min auf 800°C und Halten dieser Temperatur für einen Zeitraum von 3 Stunden.
Die BET-Oberfläche beträgt 65 m²/g.

Beispiel 5

Man pumpt eine Lösung von 1933 g $Cu(NO_3)_2$*$3H_2O$, 188 g $AlNO_3)_3$*$9H_2O$ und 2134 g $Mn(NO_3)_2$*$4H_2O$ in 24 Liter entsalztem Wasser bei 30 °C während einer Stunde in einen Behälter, in welchen eine Lösung von 2720 g NaOH in 17 Liter Wasser vorgelegt ist. Man erhöht die Temperatur nach Abschluß der Fällung auf 90°C und altert über einen Zeitraum von 12 h. Man trennt den Niederschlag mit Hilfe einer Filternutsche ab, wäscht viermal durch Resuspendieren in jeweils 20 Liter Wasser und trocknet über Nacht bei 120°C. Die Calcinierung wird wie folgt durchgeführt: Man heizt mit einer Heizrate von 2°/min auf 600°C und beläßt 3 Stunden bei dieser Temperatur. Die BET-Oberfläche wurde zu 6 m²/g bestimmt.

Beispiel 6

Man löst 341 g $Cu(NO_3)_2$*$3H_2O$, 34 g $Al(NO_3)_3$*$9H_2O$ sowie 377 g $Mn(NO_3)_2$*$4H_2O$ in entsalztem Wasser auf 3 Liter Gesamtvolumen und erwärmt die Lösung auf 50°C. Die erhaltene Lösung wird nun während einer Stunde in einen gerührten Fällbehälter gepumpt. Gleichzeitig wird ebenfalls auf 50°C erhitzte 1.5-molare Sodalösung mit einer solchen Dosierrate zugeführt, daß der pH-Wert im Fallbehälter 6.8 ± 0.2 beträgt. Nach Beendigung der Fällung wird noch eine weitere Stunde bei 50°C gerührt. Man filtriert und wäscht den Filterkuchen. Getrocknet wird über Nacht bei 120°C. Anschließend wird wie folgt calciniert: man heizt mit einer Heizrate von 2°/min auf 600°C und beläßt 3 Stunden bei dieser Temperatur. Die BET-Oberfläche wurde zu 20 m²/g bestimmt.

Beispiel 7

Man verfährt bei der Herstellung des Katalysators wie nach Beispiel 6, suspendiert aber zusätzlich 27g pyrogene Kieselsäure (CAB-O-Sil LM 150) in der Cu-, Al- und Mn-Nitrat enthaltenden Lösung. Die BET-Oberfläche wurde zu 71 m²/g bestimmt.

Beispiel 8

Die Herstellung des Katalysators wird wiederum wie nach Beispiel 6 vorgenommen. Allerdings werden der Cu-, Al- und Mn-Nitrat enthaltenden Lösung nun 27g pyrogenes $TiO_2$ (P-25, DEGUSSA) zugesetzt. Die BET-Oberfläche wurde zu 27 m²/g bestimmt.
Die Löslichkeiten der einzelnen Metallkomponenten als Maß für die Säureresistenz der hergestellten Katalysatoren werden wie folgt bestimmt:
Man rührt 10 g des calcinierten Katalysators 10 min in 100 ml 10%-iger Essigsäure bei 20°C, filtriert und analysiert den Gehalt des Filtrats an Cu, Mn, Al und gegebenenfalls Zr.

Beispiel 9 (Anwendung)

Die Hydrieraktivität der Katalysatoren in der Suspensionshydrierung von Fettsäuren wurde wie folgt bestimmt: Ein 500-ml-Rührautoklav wird mit 3 g Katalysator und 120 g eines kommerziellen Fettalkoholgemisches mit einer mittleren C-Kettenlänge von 12 bis 18 (CONDEA Alfol 1218) beschickt. Nach der Aktivierung des Katalysators bei 200°C und einem Wasserstoffdruck von 300 bar wird die Temperatur auf 300°C erhöht, worauf man 20 g Laurinsäure zudosiert. Im Verlauf der Reaktionszeit werden dem Reaktionsgemisch Proben zur Bestimmung der Verseifungszahl entnommen. Der Umsatz ermittelt sich zu

$$U = 1-(VZ_t/VZ_O)$$

wobei die Subskripte t und O die Verseifungszahlen zu Beginn bzw. zu einem Zeitpunkt t der Reaktion kennzeichnen. Unter der Annahme einer Kinetik erster Ordnung erhält man die Geschwindigkeitskonstanten k und die Halbwertszeit $t_{1/2}$ als $t_{1/2} = \ln 2/k$.

Zum Vergleich wurde ein kommerzieller Kupferchromitkatalysator für die Hydrierung von Fettsäuren (Handelsprodukt G-99B der Anmelderin; Metallgehalte: 36,5 % Cu, 32 % Cr, 2,2% Ba, 2,4% Mn) getestet. Weiterhin wurde als Vergleichsbeispiel B der Katalysator von Beispiel 39 der EP-A 0 434 062 (27% Cu, 12% Al, 61% Zr) getestet.

Die folgende Tabelle faßt die auf die beschriebene Weise erhaltene Metallöslichkeiten weiter zusammen.

| Beispiel | Säurelöslichkeit (mg Metall/10g Kat.) | Hydrieraktivität $t_{1/2}$ (min) |
|---|---|---|
| 1 | 270 | 6,5 |
| 2 | 160 | 6,2 |
| 3 | 280 | 6,7 |
| 4 | 240 | 8,2 |
| 5 | 190 | 5,9 |
| 6 | 130 | 7,5 |
| 7 | 280 | 6,9 |
| 8 | 210 | 7,2 |
| A (Vergleich) | 300 | 8,5 |
| B ( " ) | 350 | 10,5 |

Die Ergebnisse zeigen klar, daß die erfindungsgemäßen Katalysatoren sowohl in ihrer Hydrieraktivität als auch in der Beständigkeit gegenüber Säuren dem kommerziellen Vergleichskatalysator in allen Fällen zumindest ebenbürtig sind bzw. diesen sogar übertreffen. Gleichzeitig sind sie im Gegensatz zu den derzeit großtechnisch eingesetzten Katalysatoren völlig frei von umweltschädlichen Bestandteilen, so daß weder ihre Herstellung, ihre Handhabung noch die spätere Entsorgung eine Gefährdung von Mensch oder Umwelt darstellen.

**Patentansprüche**

1. Chromfreier Katalysator für die Hydrierung von organischen Verbindungen, insbesondere von organischen Verbindungen, die die Carbonylfunktion enthalten, z.B. von Aldehyden, Ketonen sowie von Carbonsäuren bzw. deren Estern, zu den entsprechenden Alkoholen gekennzeichnet durch folgende Merkmale:

   a) seine oxidische Form entspricht im wesentlichen der Zusammensetzung

   $$Cu_aAl_bZr_cMn_dO_x$$

   wobei die folgenden Beziehungen gelten:
   $a > O$; $b > O$; $c \cong O$; $d > O$; $a > b/2$; $b > a/4$; $a > c$; $a > d$;
   und x die zur Wahrung der Elektroneutralität pro Formeleinheit erforderliche Anzahl von Sauerstoffionen bezeichnet;

b) die Metallmasse, welche durch 2-minütiges Rühren von 10 g des oxidischen Katalysators in 100 ml 10 gew.%-iger Essigsäure bei 20 °C gelöst wird beträgt maximal 400 mg.

c) die BET-Oberfläche liegt zwischen 10 und 150 m$^2$/g.

2. Chromfreier Katalysator für die Hydrierung von organischen Verbindungen, insbesondere von organischen Verbindungen, die die Carbonylfunktion enthalten, z.B. von Aldehyden, Ketonen sowie von Carbonsäuren bzw. deren Estern, zu den entsprechenden Alkoholen gekennzeichnet durch folgende Merkmale:

a) seine oxidische Form entspricht im wesentlichen der Zusammensetzung

$$Cu_aAl_bZr_cMn_dO_x$$

wobei die folgenden Beziehungen gelten:
$a > O; b = a/40$ bis $a/4; c \geqq O; d > O; a > c; a = 0,5d$ bis $0,95d$
und x die zur Wahrung der Elektroneutralität pro Formeleinheit erforderliche Anzahl von Sauerstoffionen bezeichnet;

b) die Metallmasse welche durch 2-minütiges Rühren von 10 g des oxidischen Katalysators in 100 ml 10 gew.%-iger Essigsäure bei 200 °C gelöst wird beträgt maximal 400 mg.

c) die BET-Oberfläche liegt zwischen 5 und 150 m$^2$/g.

d) die oxidische Form ist durch thermische Behandlung einer Zwischenstufe im Temperaturbereich von 450 bis 900 °C, vorzugsweise im Temperaturbereich von 500 bis 800 °C, erhältlich.

3. Katalysator nach Anspruch 1 dadurch, gekennzeichnet, daß die BET-Oberfläche zwischen 25 und 125 m$^2$/g liegt.

4. Katalysator nach Anspruch 2, dadurch gekennzeichnet daß die BET-Oberfläche zwischen 5 und 100 m$^2$/g liegt.

5. Katalysator nach einem der Ansprüche 1 bis 4, dadurch gekennzeichnet, daß er einen aciditätsvermindernden Promotor, vorzugsweise ein Erdalkalioxid oder ein Seltenerdoxid, in Mengen von 0,1 bis 20 Gew.-% (berechnet als Oxide und bezogen auf die oxidische Form des Katalysators), enthält.

6. Katalysator nach einem der Ansprüche 1 bis 3 und 5, dadurch gekennzeichnet, daß er ein inertes Trägermaterial aus der Gruppe $TiO_2$, $SiO_2$ (wenn kein $Al_2O_3$ vorliegt) und deren Gemischen enthält.

7. Die vorreduzierte Form des Katalysators nach einem der Ansprüche 1 bis 6.

8. Verwendung des Katalysators nach einem der Ansprüche 1 bis 7 zur Hydrierung von ungesättigten organischen Verbindungen.

9. Verwendung des Katalysators nach einem der Ansprüche 1 bis 7, zur Hydrierung von Aldehyden, Ketonen oder Carbonsäuren bzw. deren Estern.

10. Verwendung des Katalysators nach einem der Ansprüche 1 bis 7, zur Sumpfphasenhydrierung von Carbonsäuren insbesondere von Fettsäuren bzw. deren Gemischen mit 5 bis 24 C-Atomen und/oder deren Estern, gegebenenfalls im Gemisch mit Alkoholen, in die entsprechenden Fettalkohole.

11. Verwendung nach einem der Ansprüche 6 bis 11, dadurch gekennzeichnet, daß die im Reaktionsgemisch vorhandenen Alkohole Fettalkohole oder Gemische von Fettalkoholen mit 4 bis 24 C-Atomen darstellen.

## Claims

1. A chromium-free catalyst for the hydrogenation of organic compounds, in particular organic compounds which contain the carbonyl group, far example aldehydes, ketones and also carboxylic acids or their esters, to form the corresponding alcohols, characterised by the following features:

a) its oxidic form corresponds substantially to the composition

$$Cu_a Al_b\, Zr_c Mn_d O_x$$

wherein the following relationships apply:

$a > 0; b > 0; c \geqq 0; d > 0; a > b/2; b > a/4; a > c; a > d;$

and x denotes the number of oxygen ions required to maintain electroneutrality per formula unit;

b) the metal mass, which is dissolved by stirring for 2 minutes 10 g of the oxidic catalyst into 100 ml of 10 % by weight acetic acid at 20 °C, amounts to a maximum of 400 mg;

c) the BET surface area is between 10 and 150 $m^2/g$.

2. A chromium-free catalyst for the hydrogenation of organic compounds, in particular organic compounds which contain the carbonyl group, for example aldehydes, ketones and also carboxylic acids or their esters, to form the corresponding alcohols, characterised by the following features:

a) its oxidic form corresponds substantially to the composition

$$Cu_a Al_b\, Zr_c Mn_d O_x$$

wherein the following relationships apply:

$a > 0; b = a/40$ to $a/4; c \geqq 0; d > 0; a > C; a = 0.5d$ to $0.95d$

and x denotes the number of oxygen ions required to maintain electroneutrality per formula unit;

b) the metal mass, which is dissolved by stirring for 2 minutes 10 g of the oxidic catalyst into 100 ml of 10 % by weight acetic acid at 200°C, amounts to a maximum of 400 mg;

c) the BET surface area is between 5 and 150 $m^2/g$.

d) the oxidic form can be obtained by thermal treatment of an intermediate within the temperature range of 450 to 900° C, preferably within the temperature range of 500 to 800°C.

3. A catalyst according to Claim 1, characterised in that BET surface area is between 25 and 125 $m^2/g$.

4. A catalyst according to Claim 2, characterised in that the BET surface area is between 5 and 100 $m^2/g$.

5. A catalyst according to any one of Claims 1 to 4, characterised in that it contains an acidity-decreasing promoter, preferably an alkaline-earth oxide or a rare earth oxide, in quantities of from 0.1 to 20 % by weight (calculated as oxides and related to the oxidic form of the catalyst).

6. A catalyst according to any one of Claims 1 to 3 and 5, characterised in that it contains an inert support material from the group $TiO_2$, $SiO_2$ (if no $Al_2O_3$ is present) and mixtures thereof.

7. The prereduced form of the catalyst according to any one of Claims 1 to 6.

8. Use of the catalyst according to any one of Claims 1 to 7 for the hydrogenation of unsaturated organic compounds.

9. Use of the catalyst according to any one of Claims 1 to 7 for the hydrogenation of aldehydes, ketones or carboxylic acids or their esters.

10. Use of the catalyst according to any one of Claims 1 to 7 for the liquid phase hydrogenation of carboxylic acids, in particular fatty acids or mixtures thereof having 5 to 24 C atoms and/or their esters, optionally mixed with alcohols, into the corresponding fatty alcohols.

11. Use according to any one of Claims 6 to 10, characterised in that the alcohols present in the reaction mixture constitute fatty alcohols or mixtures of fatty alcohols having 4 to 24 C atoms.

**Revendications**

1. Catalyseur exempt de chrome pour l'hydrogénation de composés organiques, en particulier de composés organiques qui contiennent la fonction carbonyle, par exemple d'aldéhydes, de cétones, ainsi que d'acides carboxyliques, respectivement de leurs esters pour obtenir les alcools correspondants, caractérisé par les particularités ci-après :

   a) sa forme oxydée correspond essentiellement à la composition

$$Cu_aAl_bZr_cMn_dO_x$$

   dans laquelle sont valables les rapports ci-après :
   $a > 0$; $b > 0$; $c \geqq 0$; $d > 0$; $a > b/2$; $b > a/4$; $a > c$; $a > d$;
   et x caractérise le nombre d'ions oxygène requis pour garantir la neutralité électronique par unité de formule;

   b) la masse métallique que l'on dissout par agitation pendant 2 minutes de 10 g du catalyseur oxydé dans 100 ml d'acide acétique à 10%, à une température de 20°C, s'élève au maximum à 400 mg;

   c) la surface BET se situe entre 10 et 150 $m^2$/g.

2. Catalyseur exempt de chrome pour l'hydrogénation de composés organiques, en particulier de composés organiques qui contiennent la fonction carbonyle, par exemple d'aldéhydes, de cétones, ainsi que d'acides carboxyliques, respectivement de leurs esters pour obtenir les alcools correspondants, caractérisé par les particularités ci-après :

   a) sa forme oxydée correspond essentiellement à la composition

$$Cu_aAl_bZr_cMn_dO_x$$

   dans laquelle sont valables les rapports ci-après :
   $a > 0$; $b = a/40$ à $a/4$; $c \geqq 0$; $d > 0$; $a > c$; $a = 0{,}5d$ à $0{,}95d$;
   et x caractérise le nombre d'ions oxygène requis pour garantir la neutralité électronique par unité de formule;

   b) la masse métallique que l'on dissout par agitation pendant 2 minutes de 10 g du catalyseur oxydé dans 100 ml d'acide acétique à 10%, à une température de 200°C, s'élève au maximum à 400 mg;

   c) la surface BET se situe entre 5 et 150 $m^2$/g;

   d) la forme oxydée peut être obtenue par traitement thermique d'un produit intermédiaire dans le domaine de température de 450 à 900°C, de préférence dans le domaine de température de 500 à 800°C.

3. Catalyseur selon la revendication 1, caractérisé en ce que la surface BET se situe entre 25 et 125 $m^2$/g.

4. Catalyseur selon la revendication 2, caractérisé en ce que la surface BET se situe entre 5 et 100 $m^2$/g.

5. Catalyseur selon l'une quelconque des revendications 1 à 4, caractérisé en ce qu'il contient un promoteur diminuant l'acidité, de préférence un oxyde de métal alcalino-terreux ou un oxyde des terres rares, dans des quantités de 0,1 à 20% en poids (calculées comme oxydes et rapportées à la forme oxydée du catalyseur).

6. Catalyseur selon l'une quelconque des revendications 1 à 3 et 5, caractérisé en ce qu'il contient une matière de support inerte choisie parmi le groupe comprenant $TiO_2$, $SiO_2$ (lorsque aucun $Al_2O_3$ n'est présent) et leurs mélanges.

7. Forme du catalyseur selon l'une quelconque des revendications 1 à 6 ayant subi une réduction préalable.

8. Utilisation du catalyseur selon l'une quelconque des revendications 1 à 7 pour l'hydrogénation de composés organiques insaturés.

9. Utilisation du catalyseur selon l'une quelconque des revendications 1 à 7 pour l'hydrogénation d'aldéhydes, de cétones ou d'acides carboxyliques, respectivement de leurs esters.

**10.** Utilisation du catalyseur selon l'une quelconque des revendications 1 à 7 pour l'hydrogénation en phase liquide d'acides carboxyliques, en particulier d'acides gras, respectivement de leurs mélanges contenant de 5 à 24 atomes de carbone et/ou de leurs esters éventuellement en mélange avec des alcools, pour obtenir les alcools gras correspondants.

**11.** Utilisation selon l'une quelconque des revendications 6 à 10, caractérisée en ce que les alcools présents dans le mélange réactionnel représentent des alcools gras ou des mélanges d'alcools gras contenant de 4 à 24 atomes de carbone.